# EUROPÄISCHE PATENTANMELDUNG

(11) **EP 1 985 321 A1**
(43) Veröffentlichungstag der Anmeldung: **29.10.2008**
(21) Anmeldenummer: 08103725.1
(22) Anmeldetag: 25.04.2008
(51) Int. Cl.: A61M 5/142, A61M 5/168, F15D 1/14, F16L 55/027

(54) **Pumpe, Verfahren zur Herstellung und Verfahren zur Inbetriebnahme der Pumpe**

(30) Priorität: 26.04.2007 DE 102007020163
(71) Anmelder: Tricumed Medizintechnik GmbH, 24143 Kiel (DE)
(72) Erfinder: Dipl.-Ing. Otto,, Karl-Heinz, 24146, Kiel (DE); Dr. Zacharias,, Volker, 24106, Kiel (DE)
(74) Vertreter: Schäfer, Horst

(57) **Zusammenfassung**

Eine Pumpe weist ein Gehäuse (19) und einen innerhalb des Gehäuses (19) angeordneten Vorratsraum zur Aufnahme und Vorhaltung einer Flüssigkeit und ferner eine ebenfalls in dem Gehäuse (19) angeordnete Drosseleinheit mit einer Drosselstrecke auf. Die Drosseleinheit umfasst die Kanalplatte (1) mit einer ersten Seite (2) und einer zweiten Seite (3), einem in die erste Seite (2) der Kanalplatte (1) eingebrachten, die Drosselstrecke ausbildenden Kanal (4) und mit einer Anzahl von einen Boden des Kanals (4) mit der zweiten Seite (3) der Kanalplatte (1) durch einen Fließweg verbindenden ersten Durchflussöffnungen (5). Sie umfasst ferner eine Deckelplatte (15) mit einer die zweite Seite (17) mit der ersten Seite (16) durch einen Fließweg verbindenden zweiten Durchflussöffnung (18), wobei die zweite Seite (17) der Deckelplatte (15) an der ersten Seite (2) der Kanalplatte (1) anliegt. Sie umfasst ferner eine Dichtungsplatte (11) mit einer deren erste Seite (12) mit deren zweiter Seite (13) durch einen Fließweg verbindenden dritten Durchflussöffnung (14), wobei die erste Seite (12) der Dichtungsplatte (11) an der zweiten Seite (3) der Kanalplatte (1) anliegt. Der Kanal (4) weist einen ersten Kanalabschnitt (9) zwischen einem Ende (6) des Kanals (4) und einer vorderen ersten Durchflussöffnung (7) durch die Kanalplatte (1) und eine Anzahl von sich in einer Fließrichtung der Flüssigkeit hintereinander an den ersten Kanalabschnitt (9) anschließenden Verlängerungsabschnitten (10) auf.

## Beschreibung

Die Erfindung betrifft eine Pumpe, insbesondere eine zum Fördern besonders geringer Flüssigkeitsmengen geeignete Pumpe wie beispielsweise eine in den Körper eines Patienten implantierbare Infusionspumpe. Sie betrifft ferner ein Verfahren zur Herstellung und ein Verfahren zur Inbetriebnahme der Pumpe.

Zur Förderung und Dosierung der Abgabe geringer Flüssigkeitsmengen geeignete Pumpen wie beispielsweise implantierbare Infusionspumpen weisen ein Gehäuse auf, in dem ein Medikamentenraum zur Aufnahme und Vorhaltung eines Medikaments, beispielsweise eines Schmerzmittels, angeordnet ist. Bei Infusionspumpen, die besonders einfach aufgebaut sind und keine elektronische Regelung der Flussrate aufweisen, erfolgt die Abgabe des Medikaments über eine zu einem im Körper des Patienten mündenden Katheter führende Drosselstrecke, wobei die Dosierung des Medikaments über die Geometrie, insbesondere über Länge und Durchmesser, der Drosselstrecke eingestellt wird.

Aus der EP 0 369 712 A2 ist eine Drosselstrecke für eine Infusionspumpe bekannt, die jedoch verhältnismäßig kompliziert aufgebaut ist. Eine derartige Drosselstrecke ist einer Miniaturisierung, wie sie für den Bau implantierbarer Infusionspumpen notwendig ist, nicht ohne Weiteres zugänglich.

Eine implantierbare Infusionspumpe ist beispielsweise aus der EP 0 189 940 A2 bekannt. Sie weist eine mäandrisch geformte Drosselstrecke auf, die als Kanal in einen Siliziumchip geätzt wurde. Dies hat den Vorteil, dass aus der Halbleitertechnologie bekannte und bewährte Verfahren der Photolithographie und Ätztechnik zum Einsatz kommen und sehr feine Strukturen hergestellt werden können. Auf diese Weise lassen sich Drosselstrecken mit einer großen Länge herstellen, was nach dem Gesetz von Hagen-Poiseuille einem geringen Volumendurchsatz entspricht. Bei der Infusionspumpe gemäß der EP 0 189 940 A2 ist es nicht möglich, die Förderrate individuell einzustellen.

Aus der EP 0 961 624 B1 ist es bekannt, in einer einzigen Kanalplatte einer Infusionspumpe gleich mehrere unterschiedlich lange Kanäle unterzubringen. Die unterschiedlichen Längen der Kanäle bedingen unterschiedliche Förderraten der Pumpe. Bevor die Infusionspumpe implantiert wird, wird der zur gewünschten Förderrate passende Kanal ausgewählt und durch eine entsprechende Montage "aktiviert". Dabei ist nachteilig, dass die Kanalplatte für eine Verwirklichung möglichst vieler unterschiedlicher Förderraten verhältnismäßig groß ausgebildet sein muss.

Aufgabe der Erfindung ist es daher, eine zur Förderung kleiner Flüssigkeitsmengen geeignete Pumpe anzugeben, die eine möglichst individuelle Einstellung der Förderrate erlaubt, gleichzeitig aber möglichst kompakt ist. Eine weitere Aufgabe ist es, Verfahren zur Herstellung und zur Inbetriebnahme einer solchen Pumpe anzugeben.

Erfindungsgemäß wird diese Aufgabe mit dem Gegenstand der unabhängigen Patentansprüche gelöst. Vorteilhafte Weiterbildungen der Erfindung sind Gegenstand der abhängigen Patentansprüche.

Eine Pumpe weist erfindungsgemäß ein Gehäuse und einen innerhalb des Gehäuses angeordneten Vorratsraum zur Aufnahme und Vorhaltung einer Flüssigkeit auf. Ebenfalls in dem Gehäuse angeordnet ist eine Drosseleinheit mit einer die Abgabe der Flüssigkeit dosierenden, zu einem Pumpenauslass führenden Drosselstrecke. Die Drosselstrecke ist als in eine erste Seite einer Kanalplatte eingebrachter Kanal ausgebildet, wobei der Kanal einen ersten Kanalabschnitt zwischen einem Ende des Kanals und einer vorderen ersten Durchflussöffnung durch die Kanalplatte und eine Anzahl von sich in einer Fließrichtung oder entgegengesetzt einer Fließrichtung der Flüssigkeit hintereinander an den ersten Kanalabschnitt anschließenden Verlängerungsabschnitten zwischen der vorderen ersten Durchflussöffnung und weiteren ersten Durchflussöffnungen durch die Kanalplatte aufweist.

Die Drosseleinheit umfasst die Kanalplatte mit der ersten Seite und einer zweiten Seite, dem in die erste Seite der Kanalplatte eingebrachten Kanal und mit einer Anzahl von einen Boden des Kanals mit der zweiten Seite der Kanalplatte durch einen Fließweg verbindenden ersten Durchflussöffnungen.

An der ersten Seite der Kanalplatte liegt der die Drosselstrecke bildende Kanal frei. Um eine allseitig geschlossene Fluidbahn für die Flüssigkeit zu bilden, wird der Kanal mit einer Deckelplatte abgedeckt.

Die Drosseleinheit umfasst demnach auch eine Deckelplatte mit einer ersten Seite und einer zweiten Seite. Eine zweite Durchflussöffnung in der Deckelplatte verbindet die zweite Seite mit der ersten Seite durch einen Fließweg. Die Deckelplatte ist derart montiert, dass ihre zweite Seite an der ersten Seite der Kanalplatte anliegt.

Die Drosseleinheit umfasst ferner eine Dichtungsplatte mit einer ersten Seite und einer zweiten Seite. Eine dritte Durchflussöffnung verbindet die erste Seite mit der zweiten Seite durch einen Fließweg. Die Dichtungsplatte ist derart montiert, dass ihre erste Seite an der zweiten Seite der Kanalplatte anliegt.

Diese Pumpe hat den Vorteil, dass mehrere unterschiedliche Kanallängen und damit Förderraten in einem einzigen Kanal verwirklich sind. Die geringste Länge und damit die höchste Förderrate wird erzielt, wenn lediglich das Kanalstück zwischen dem einen Ende des Kanals und der ersten Durchflussöffnung als Drosselstrecke genutzt wird. In diesem Fall ist die Kanalplatte so montiert, dass die erste Durchflussöffnung als Einlass oder als Auslass für die Flüssigkeit fungiert. Die Drosselstrecke kann jedoch bei Bedarf um einen oder mehrere Verlängerungsabschnitte verlängert werden, was jeweils die Förderrate der Pumpe senkt. Pro weiterer einstellbarer Förderrate ist lediglich ein weiterer Verlängerungsabschnitt erforderlich, nicht aber ein ganz eigener Kanal, da eine längere Drosselstrecke jeweils die kürzeren beinhaltet. Durch die Anbringung zahlreicher weiterer erster Durchflussöffnungen kann zwischen einer minimalen und einer maximalen Förderrate eine nahezu beliebig feine Einstellung der Förderrate ermöglicht werden, ohne dass der Platzbedarf für die Drosselstrecke steigt.

In einer Ausführungsform ist die Dichtungsplatte bewegbar und weist mehrere Betriebsstellungen auf, wobei die dritte Durchflussöffnung in jeder Betriebsstellung mit einer der ersten Durchflussöffnungen in der Kanalplatte fluchtet. Dies hat den Vorteil, dass die Kanalplatte mit bereits montierter Dichtungsplatte oder noch zu montierender Dichtungsplatte ausgeliefert und die Betriebsstellung der Dichtungsplatte unmittelbar vor der vollständigen Montage, im Falle einer implantierbaren Infusionspumpe unmittelbar vor der Implantation, festgelegt werden kann.

Dies ist insbesondere vorteilhaft bei Pumpen wie implantierbaren Infusionspumpen, die in verhältnismäßig kleiner Stückzahl hergestellt, jedoch mit den verschiedensten Förderraten eingesetzt werden. Statt einzelne Kanalplatten mit individuellen Förderraten herzustellen, was sehr aufwendig und damit kostenintensiv wäre, können für eine Vielzahl von Förderraten gleichzeitig taugliche Kanalplatten hergestellt werden, weil erst bei der Montage und nur durch die Wahl einer entsprechenden Betriebsstellung der Dichtungsplatte die individuelle Förderrate eingestellt werden kann.

In einer Ausführungsform der Erfindung ist die dritte Durchflussöffnung größer als die ersten Durchflussöffnungen. Das hat den Vorteil, dass die ersten Durchflussöffnungen derart angeordnet werden können, dass in jeder Betriebsstellung der Dichtungsplatte die dritte Durchflussöffnung mit genau einer der ersten Durchflussöffnungen fluchtet.

In einer Ausführungsform der Erfindung ist die Dichtungsplatte drehbar. Dazu kann sie beispielsweise um ihre Mittelachse rotierbar an der Kanalplatte montiert sein, so dass jeder Betriebsstellung der Dichtungsplatte ein bestimmter Winkel entspricht, um den die Dichtungsplatte gegen eine Ausgangsstellung verdreht wird. Die ersten Durchflussöffnungen sind dann derart angeordnet, dass für jeden der Winkel genau eine der ersten Durchflussöffnungen in der dritten Durchflussöffnung frei liegt.

In einer alternativen Ausführungsform ist die Dichtungsplatte zwischen mehreren Betriebsstellungen verschiebbar angeordnet.

In einer vorteilhaften Ausführungsform der Erfindung weist der Kanal eine Spiralform auf, insbesondere kann der Kanal die Form einer archimedischen Spirale aufweisen. Es sind jedoch auch andere, beispielsweise mäanderförmige Strukturen denkbar, die den auf der Kanalplatte vorhandenen Platz möglichst gut zur Bildung der Drosselstrecke ausnutzen. Die Spiralform des Kanals hat den Vorteil, dass ein besonders gleichmäßiger Fluss der Flüssigkeit durch die Drosselstrecke möglich ist. Bei mäandrisch verlaufenden Drosselstrecken besteht dagegen eher die Gefahr, dass, insbesondere bei langen Molekülen, im Totwassergebiet der Ecken Bestandteile der Flüssigkeit auskristallisieren oder sich ablagern.

In einer Ausführungsform der Erfindung weist die Kanalplatte ein mikrostrukturierbares Glas auf. Die Kanalplatte kann jedoch zusätzlich oder anstelle des mikrostrukturierbaren Glases auch beispielsweise Silizium aufweisen.

Mikrostrukturierbares Glas ist fotoempfindlich und sehr einfach mit photolithografischen Methoden strukturierbar. Es weist zudem auch eine hohe Beständigkeit gegenüber basischen Lösungen auf. Dies ist vorteilhaft, wenn die Pumpe auch zur Förderung Flüssigkeiten, beispielsweise mit einem pH-Wert ≥ 10 eingesetzt werden soll. Medizinische Trägerflüssigkeiten weisen teilweise derart hohe pH-Werte auf. Die Kanalplatte kann vollständig aus fotoempfindlichem Glas ausgebildet oder ihre erste Seite lediglich damit beschichtet sein.

Unter einem fotoempfindlichen oder mikrostrukturierbaren Glas wird hier und im folgenden ein Glas wie beispielsweise Foturan verstanden, das durch Belichtung mit Photonen einer bestimmten Wellenlänge seine Struktur verändert und in den belichteten Bereichen Nanokristalle oder andere Kristallisationskeime ausbildet. Diese Kristallisationskeime können durch Wärmezufuhr zu einer Kristallstruktur wachsen, die für ein Ätzmittel wesentlich leichter angreifbar ist als die noch amorphen Bereiche des Glases. Folglich können die kristallinen Bereiche in einem Ätzprozess leicht entfernt werden.

Der Einsatz von Silizium für die Kanalplatte hat als Standardmaterial in der Halbleitertechnologie den Vorteil, dass es preiswert ist und mit bewährten Verfahren verarbeitet werden kann. Um es vor dem Einfluss basischer Lösungen zu schützen, kann für alle Oberflächen der Kanalplatte, die mit der Flüssigkeit in Kontakt kommen, ein anderes, gegenüber basischen Lösungen resistentes Material vorgesehen sein.

Der Querschnitt des Kanals ist in einer Ausführungsform der Erfindung im Wesentlichen rechteckig. Ein Kanal mit rechteckigen Querschnitt entsteht durch eine gleichmäßige Belichtung und damit eine gleichmäßige Eindringtiefe des Lichts in das Glas.

Die Deckelplatte ist beispielsweise aus Glas ausgebildet. Dabei muss es sich nicht notwendig um mikrostrukturierbares Glas handeln, da in die Deckelplatte außer der zweiten Durchflussöffnung keine Strukturen eingebracht werden müssen. Glas als Material für die Deckelplatte hat den Vorteil, dass die Deckelplatte mit ihrer zweiten Seite fest auf die erste Seite der Kanalplatte aufgebondet sein kann und somit die Kanalstruktur sicher abdeckt.

Die Dichtungsplatte ist in einer vorteilhaften Ausführungsform aus Silikon ausgebildet. Das hat den Vorteil, dass die sämtlichen ersten Durchflussöffnungen, die in der gewählten Betriebsstellung nicht in der dritten Durchflussöffnung frei liegen, durch die Dichtungsplatte vollständig und zuverlässig abgedichtet werden.

Die Kanalplatte mit auf die erste Seite aufgebondeter Deckelplatte und an der zweiten Seite anliegender Dichtungsplatte kann auf unterschiedliche Weise in dem Gehäuse der Pumpe montiert sein. Insbesondere ist es grundsätzlich möglich, den durch die Durchflussöffnungen und den Kanal gebildeten Fließweg in beiden Richtungen von der Flüssigkeit durchströmen zu lassen.

In einer ersten Ausführungsform der Erfindung ist die zweite Durchflussöffnung in der Deckelplatte in Fließrichtung der Flüssigkeit vor der dritten Durchflussöffnung in der Dichtungsplatte angeordnet, so dass durch einen Einlass in einem Verschluss des Vorratsraums, durch die zweite Durchflussöffnung in der Deckelplatte, durch den Kanal, durch eine erste Durchflussöffnung in der Kanalplatte und durch die dritte Durchflussöffnung in der Dichtungsplatte ein Fließweg für die Flüssigkeit gebildet ist.

Bei dieser Ausführungsform ist die erste Seite der Deckelplatte vorteilhafterweise gegen einen Verschluss des Vorratsraums mittels eines O-Ringes aus einem gummielastischen Material abgedichtet.

Dazu können in dem Verschluss des Vorratsraums Ausnehmungen zur Aufnahme des O-Rings angeordnet sein.

In einer zweiten Ausführungsform der Erfindung ist die zweite Durchflussöffnung in der Deckelplatte in Fließrichtung der Flüssigkeit nach der dritten Durchflussöffnung in der Dichtungsplatte angeordnet, so dass durch einen Einlass in einem Verschluss des Vorratsraums, durch die dritte Durchflussöffnung in der Dichtungsplatte, durch eine erste Durchflussöffnung in der Kanalplatte, durch den Kanal und durch die zweite Durchflussöffnung in der Deckelplatte ein Fließweg für die Flüssigkeit gebildet ist.

Bei dieser Ausführungsform ist vorteilhafterweise die erste Seite der Deckelplatte gegen das Gehäuse mittels eines O-Ringes aus einem gummielastischen Material abgedichtet.

Dazu können im Gehäuse Ausnehmungen zur Aufnahme des O-Rings angeordnet sein.

In dem Gehäuse ist vorteilhafterweise ein Treibmittelraum zur Aufnahme eines verdampfbarem Treibmittels angeordnet. Das verdampfende Treibmittel stellt die zum Austreiben der Flüssigkeit durch die Drosselstrecke erforderliche Kraft zur Verfügung. Grundsätzlich sind jedoch auch andere Mechanismen zur Bereitstellung dieser Kraft denkbar.

Die Pumpe ist in einer Ausführungsform als Infusionspumpe zum Befördern eines Medikaments aus dem Vorratsraum in den Körper eines Patienten ausgebildet, insbesondere als in den Körper des Patienten implantierbare Infusionspumpe.

Ein erfindungsgemäßes Verfahren zur Herstellung einer Kanalplatte mit einer Drosselstrecke für eine Pumpe weist folgende Schritte auf: Es wird eine Kanalplatte mit einer ersten Seite und einer zweiten Seite bereitgestellt und ein Kanal in die erste Seite der Kanalplatte, beispielsweise durch Ätzen, eingebracht. Ferner werden erste Durchflussöffnungen in die Kanalplatte eingebracht, die den Boden des Kanals mit der zweiten Seite der Kanalplatte durch einen Fließweg verbinden. Auf die erste Seite der Kanalplatte wird eine Deckelplatte, beispielsweise aus Glas, mit ihrer zweiten Seite aufgebondet, wobei eine zweite Durchflussöffnung in der Deckelplatte mit einem Beginn des Kanals fluchtet. Auf die zweite Seite der Kanalplatte wird eine Dichtungsplatte mit ihrer ersten Seite aufgebracht. Abschließend wird die Kanalplatte mit aufgebondeter Deckelplatte und Dichtungsplatte in ein Gehäuse eingesetzt.

Ein erfindungsgemäßes Verfahren zur Inbetriebnahme einer Pumpe beinhaltet das Einstellen einer Flussrate der Flüssigkeit durch den Kanal dadurch, dass eine der ersten Durchflussöffnungen ausgewählt wird, für die der Fließweg der Flüssigkeit im Kanal und damit die Förderrate der Pumpe eine gewünschte Länge aufweist, und die dritte Durchflussöffnung derart positioniert wird, dass sie mit der ausgewählten ersten Durchflussöffnung fluchtet und die Dichtungsplatte sämtliche anderen ersten Durchflussöffnungen abdeckt.

Dieses Verfahren hat den Vorteil, dass die Flussrate über die Länge der Drosselstrecke individuell auf den Einsatzzweck der Pumpe abgestimmt werden kann, und zwar erst im Zeitpunkt des Einsatzes, nicht bereits bei der Herstellung der Kanalplatte. Dies ist insbesondere vorteilhaft bei implantierbaren Infusionspumpen, die besonders klein und leicht ausgeführt werden sollen.

Ausführungsbeispiele der Erfindung werden im folgenden anhand der beigefügten Figuren näher erläutert.
- Figur 1: zeigt schematisch eine perspektivische Ansicht eines Ensembles aus einer Kanalplatte, einer Deckelplatte und einer Dichtungsplatte gemäß einem Ausführungsbeispiel der Erfindung;
- Figur 2: zeigt schematisch eine weitere perspektivische Ansicht des Ensembles gemäß Figur 1;
- Figur 3: zeigt schematisch eine Untersicht auf eine Kanalplatte mit aufgesetzter Dichtungsplatte gemäß einem Ausführungsbeispiel der Erfindung;
- Figur 4: zeigt schematisch eine perspektivische Ansicht der Kanalplatte mit aufgesetzter Deckelplatte gemäß Figur 3;
- Figur 5: zeigt schematisch im Querschnitt eine implantierbare Infusionspumpe gemäß einem Ausführungsbeispiel der Erfindung.

Gleiche Teile sind in allen Figuren mit den gleichen Bezugszeichen versehen.

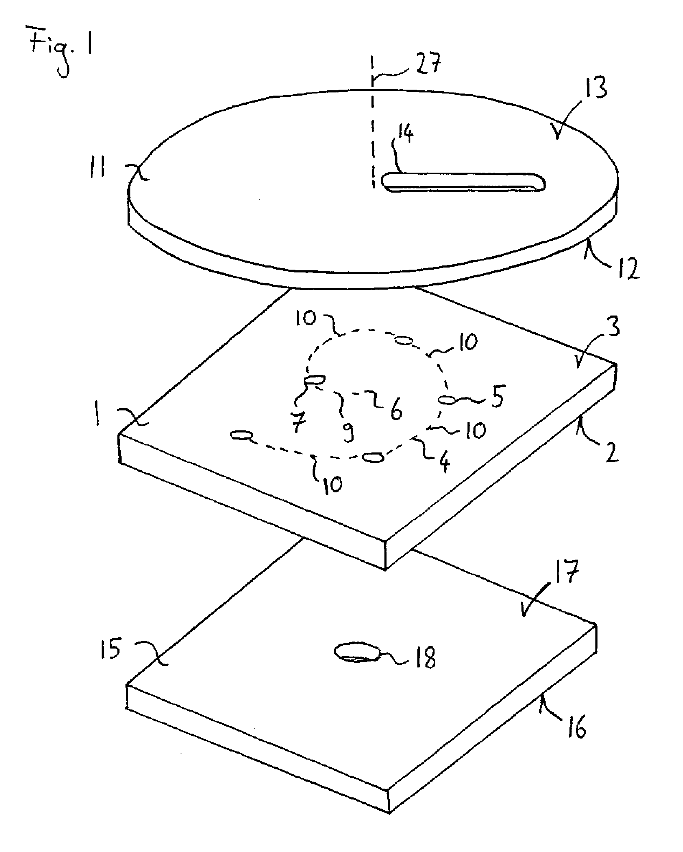

Figur 1 zeigt schematisch eine Explosionsansicht eines Ensembles aus einer Kanalplatte 1, einer Deckelplatte 15 und einer Dichtungsplatte 11 einer Drosseleinheit einer nicht dargestellten Pumpe gemäß einem Ausführungsbeispiel der Erfindung. Die zwischen Deckelplatte 15 und Dichtungsplatte 11 angeordnete Kanalplatte 1 weist eine erste Seite 2 und eine zweite Seite 3 auf. In die erste Seite 2 ist ein Kanal 4 eingebracht, der einen Fließweg für eine Flüssigkeit bereitstellt und eine Drosselstrecke für die Pumpe bildet. Die Kanalplatte 1 ist in dieser Ausführungsform aus einem fotostrukturierbaren Glas ausgebildet.

In der gezeigten Ansicht ist der Kanal 4 lediglich durch eine gestrichelte Linie angedeutet, da er in die erste Seite 2 der Kanalplatte 1 eingebracht ist, die nicht sichtbar ist. An der sichtbaren zweiten Seite 3 der Kanalplatte 1 liegen erste Durchflussöffnungen 5 frei, die den Boden des Kanals 4 mit der zweiten Seite 3 durch einen Fließweg verbinden. Zwischen der einem Beginn bzw. Ende 6 des Kanals in Fließrichtung einer Flüssigkeit nächstgelegene erste Durchflussöffnung 5, die als vordere erste Durchflussöffnung 7 bezeichnet wird, und dem Ende 6 des Kanals 4 liegt ein erster Kanalabschnitt 9. An den ersten Kanalabschnitt 9 schließen sich eine Anzahl von Verlängerungsabschnitten 10 an, die jeweils zu weiteren ersten Durchflussöffnungen 5 führen.

Der Kanal 5 kann in beide Richtungen durchflossen werden. Die Bezeichnung "Ende 6" des Kanals 4 wird daher hier allgemein verwendet. Der durch sie bezeichnete Gegenstand kann sowohl die Rolle eines Einlasses für eine Flüssigkeit in den Kanal als auch die Rolle eines Auslasses spielen. Im ersten Fall sind die ersten Durchflussöffnungen 5 dem Ende 6 des Kanals in Fließrichtung vorgeschaltet, im zweiten Fall nachgeschaltet.

Der erste Kanalabschnitt 9 stellt in jedem Fall einen Teil des Fließwegs der Flüssigkeit dar. Je nach gewünschter Förderleistung der Pumpe kann der Fließweg jedoch noch durch weitere, sich direkt an den ersten Kanalabschnitt 9 anschließende Verlängerungsabschnitte 10 verlängert werden.

Dazu wird mit Hilfe der Dichtungsplatte 11 eine der weiteren ersten Durchöffnungen 5 ausgewählt, die die Länge des Fließwegs festlegt. Dies geschieht folgendermaßen:

Die Dichtungsplatte weist eine erste Seite 12 und eine zweite Seite 13 auf und eine dritte Durchflussöffnung 14, die die erste Seite 12 mit der zweiten Seite 13 durch einen Fließweg verbindet. Die Dichtungsplatte 11 wird auf die Kanalplatte 4 aufgesetzt, so dass ihre erste Seite 12 an der zweiten Seite 3 der Kanalplatte 4 anliegt. Die Dichtungsplatte 11 ist gegenüber der Kanalplatte 4 beweglich, und zwar ist sie in dem gezeigten Ausführungsbeispiel um ihre Mittelachse 27 drehbar.

Bei der Inbetriebnahme der Pumpe wird die der gewünschten Förderrate entsprechende Fließweglänge durch die Auswahl einer ersten Durchflussöffnung 5 eingestellt. Die Auswahl der ersten Durchflussöffnung 5 erfolgt durch Drehung der Dichtungsplatte 11 um ihre Mittelachse 27 solange, bis die dritte Durchflussöffnung 14 mit der auszuwählenden ersten Durchflussöffnung 5 fluchtet. In dieser ausgewählten Betriebsstellung der Dichtungsplatte 11 wird diese fixiert, beispielsweise durch endgültiges Aufsetzen auf die Kanalplatte 1. Die Dichtungsplatte 11 deckt dann alle anderen ersten Durchflussöffnungen 5 ab, so dass der Fließweg vom Ende 6 des Kanals zu der ausgewählten ersten Durchflussöffnung 5 festgelegt ist. Damit eine sichere, dichte Abdeckung der nicht ausgewählten ersten Durchflussöffnungen 5 gewährleistet ist, ist die Dichtungsplatte 11 in diesem Ausführungsbeispiel aus Silikon, beispielsweise als Silikonflachdichtung, ausgeführt.

Die Drosseleinheit weist weiterhin eine Deckelplatte 15 mit einer ersten Seite 16 und einer zweiten Seite 17 auf. Die Deckelplatte 15 liegt im montierten Zustand mit ihrer zweiten Seite 17 an der ersten Seite 2 der Kanalplatte 1 an. Die Deckelplatte 15, die in diesem Ausführungsbeispiel aus Glas besteht und auf die Kanalplatte aufgebondet ist, weist eine zweite Durchflussöffnung 18 auf, die die erste Seite 16 mit der zweiten Seite 17 durch einen Fließweg verbindet. Im montierten Zustand fluchtet die zweite Durchflussöffnung 18 mit dem Ende 6 des Kanals 4, so dass ein Fließweg für eine Flüssigkeit durch die zweite Durchflussöffnung 18, durch den Kanal 4, durch die ausgewählte erste Durchflussöffnung 5 und durch die dritte Durchflussöffnung 14 gebildet ist.

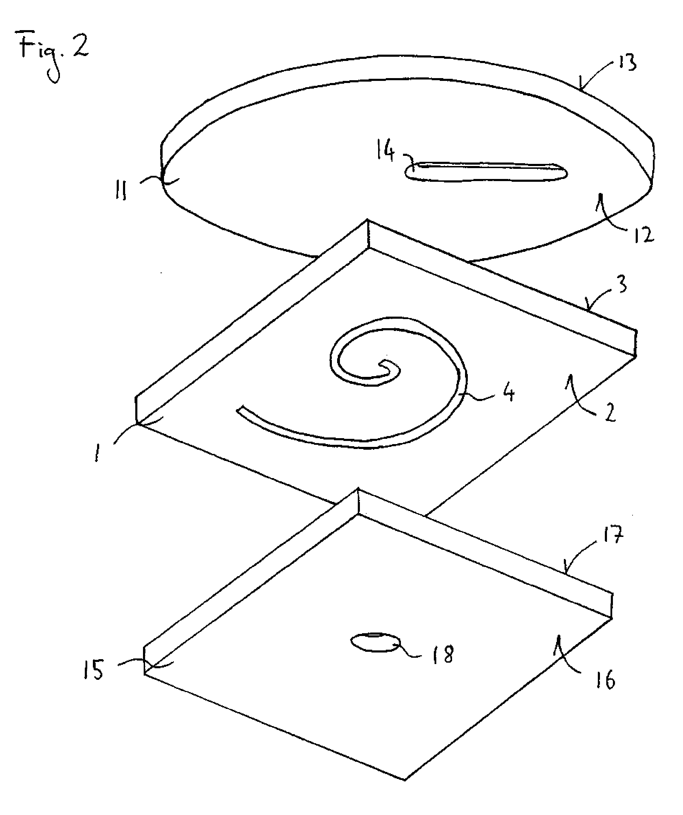

Figur 2 zeigt das Ensemble gemäß Figur 1 in einer Ansicht schräg von unten, so dass anstelle der zweiten Seiten von Deckelplatte 15, Kanalplatte 1 und Dichtungsplatte 11 deren erste Seiten sichtbar sind. In dieser Ansicht ist auch der Kanal 4 in der ersten Seite 2 der Kanalplatte 1 sichtbar.

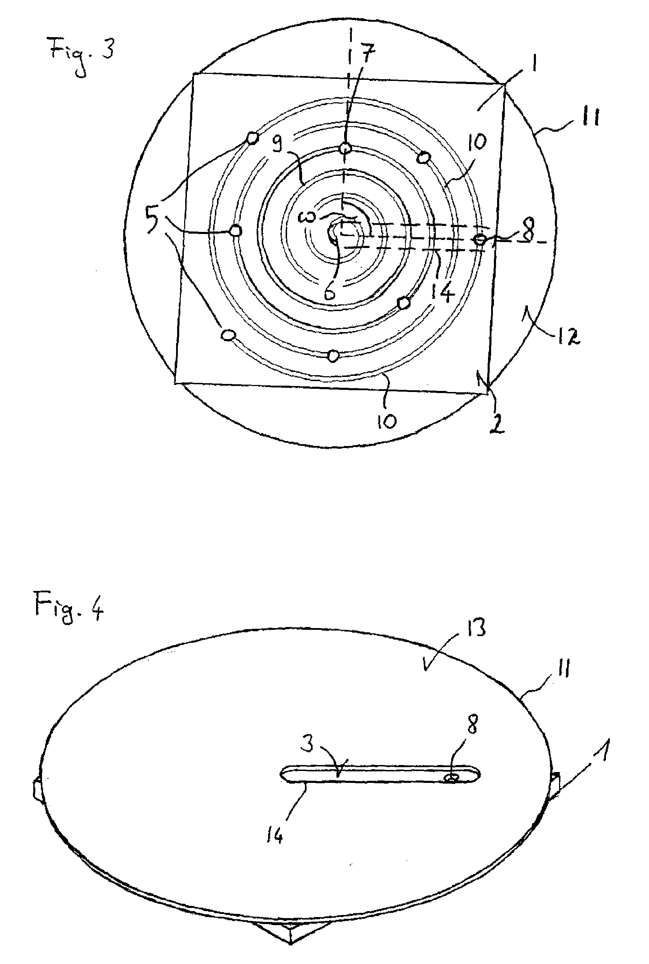

Figur 3 zeigt eine Sicht auf die erste Seite 2 der Kanalplatte 1, wobei auf die zweite Seite 3 der Kanalplatte 1 die Dichtungsplatte 11 aufgesetzt ist. In die erste Seite 2 der Kanalplatte ist der Kanal 4 eingebracht, der in diesem Ausführungsbeispiel die Form einer archimedischen Spirale aufweist.

Wie in dieser Darstellung besonders gut erkennbar ist, wird durch die Wahl einer Betriebsstellung für die Dichtungsplatte 11, die in diesem Ausführungsbeispiel durch den Winkel ω gekennzeichnet ist, eine der ersten Durchflussöffnungen 5 ausgewählt, so dass diese mit der dritten Durchflussöffnung 14, die in dieser Ansicht nicht sichtbar und daher nur getrichelt angedeutet ist, fluchtet. Die die Förderrate der Pumpe durch ihre Länge bestimmende Drosselstrecke erstreckt sich zwischen dem Ende 6 des Kanals 4 und der ausgewählten ersten Durchflussöffnung 8. In diesem Ausführungsbeispiel ist in jeder Betriebsstellung der Dichtungsplatte 11 und damit jeweils einem Winkel ω nur eine erste Durchflussöffnung 8 ausgewählt.

In der Darstellung in Figur 4 ist besonders gut sichtbar, dass in einer Betriebsstellung der Dichtungsplatte 11 diese alle ersten Durchflussöffnungen 5 bis auf die eine ausgewählte erste Durchflussöffnung 8 abdeckt.

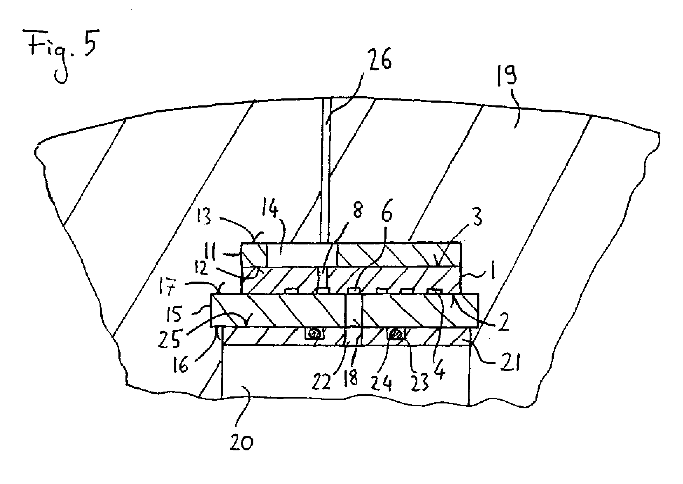

Figur 5 zeigt schematisch einen Querschnitt durch eine implantierbare Infusionspumpe gemäß einem Ausführungsbeispiel der Erfindung. Die Kanalplatte 1 ist zusammen mit der Deckelplatte 15 und der Dichtungsplatte 11 benachbart zu einem Verschluss 21 eines Vorratsraums oder Medikamentenraums 20 der Infusionspumpe angeordnet. Zusammen sind sie in ein Gehäuse 19 der Infusionspumpe eingebettet.

In den Ausführungsbeispiel gemäß Figur 5 ist die Deckelplatte 15 aus Glas ausgebildet und so auf die Kanalplatte 1 aufgebondet, dass eine zweite Seite 17 der Deckelplatte 15 an der ersten Seite 2 der Kanalplatte 1 anliegt. Die zweite Seite 17 der Deckelplatte 15 bildet somit die fehlende vierte Wand des Kanals 4.

Ein Durchlass 22 in dem Verschluss 21 fluchtet mit der zweiten Durchflussöffnung 18 durch die Deckelplatte 15. Durch den Durchlass 22 und die Durchflussöffnung 18 durch die Deckelplatte 15 ist ein Fließweg für ein Medikament aus dem Medikamentenraum 20 zum Ende 6 des Kanals 4 gebildet. Ein im Medikamentenraum 20 vorgehaltenes Medikament tritt beispielsweise aufgrund des Druckes eines verdampfenden Treibmittels in einem nicht dargestellten Treibmittelraum durch diesen Fließweg in den Kanal 4 ein. Der Kanal 4 fungiert als Drosselstrecke für die Infusionspumpe und sein Fließwiderstand, der maßgeblich durch seinen Querschnitt und seine Länge bestimmt wird, dosiert die Abgabe des Medikaments.

Das Medikament durchfließt den Kanal 4 und tritt durch eine ausgewählte erste Durchflussöffnung 8 durch die Kanalplatte 1 und die dritte Durchflussöffnung 14 in der Dichtungsplatte 11 und schließlich durch einen mit der dritten Durchflussöffnung 14 fluchtenden Pumpenauslass 26 in dem Gehäuse 19 aus, von wo aus es an seinen Wirkort in den Körper eines Patienten gelangt.

Die Deckelplatte 15 ist gegen den Verschluss 21 durch eine Dichtung aus einem O-Ring 24 beispielsweise aus einem gummielastischen Material abgedichtet. Dazu ist in dem Verschluss 21 eine ringförmige Ausnehmung 23 vorgesehen, in der der O-Ring 21 aus einem gummielastische Material angeordnet werden kann. Ähnliche Dichtungen können bei Bedarf auch zwischen anderen Teilen der Infusionspumpe vorgesehen sein.

In einem nicht dargestellten Ausführungsbeispiel ist die Drosseleinheit umgekehrt in dem Gehäuse 19 eingebaut, so dass die zweite Seite der Dichtungsplatte 11 an der Oberseite 25 des Verschlusses 21 anliegt. In diesem Fall verläuft der Fließweg des Medikaments umgekehrt, nämlich durch den Durchlass 22, durch die dritte Durchflussöffnung 14, durch die ausgewählte erste Durchflussöffnung 8, den Kanal 4, durch die zweite Durchflussöffnung 18 und schließlich durch den Pumpenauslass 26. Die Deckelplatte 15 ist dann gegen das Gehäuse 19 mittels eines O-Ringes abgedichtet.

Signifikante Gegenstände der Erfindung sind auch
- eine Pumpe, wobei die Dichtungsplatte (11) drehbar ist.
- eine Pumpe, wobei die Dichtungsplatte (11) verschiebbar ist.
- eine Pumpe, wobei der Kanal (4) eine Spiralform aufweist.
- eine Pumpe, wobei der Kanal (4) die Form einer archimedischen Spirale aufweist.
- eine Pumpe, wobei der Kanal (4) einen rechteckigen Querschnitt aufweist.
- eine Pumpe, wobei in dem Verschluss (21) des Vorratsraums Ausnehmungen (23) zur Aufnahme des O-Rings (24) angeordnet sind.
- eine Pumpe, wobei im Gehäuse (19) Ausnehmungen zur Aufnahme des O-Rings (19) angeordnet sind.
- eine Pumpe, wobei in dem Gehäuse (19) ein Treibmittelraum zur Aufnahme eines verdampfbarem Treibmittels angeordnet ist.
- eine Pumpe, die als Infusionspumpe zum Befördern eines Medikaments aus dem Vorratsraum in den Körper eines Patienten ausgebildet ist.

## Patentansprüche

1. Pumpe mit einem Gehäuse (19) und einem innerhalb des Gehäuses (19) angeordneten Vorratsraum zur Aufnahme und Vorhaltung einer Flüssigkeit und einer ebenfalls in dem Gehäuse (19) angeordneten Drosseleinheit mit einer die Abgabe der Flüssigkeit dosierenden, zu einem Pumpenauslass (26) führenden Drosselstrecke,
wobei die Drosseleinheit folgendes umfasst:
- eine Kanalplatte (1) mit einer ersten Seite (2) und einer zweiten Seite (3), einem in die erste Seite (2) der Kanalplatte (1) eingebrachten, die Drosselstrecke ausbildenden Kanal (4) und mit einer Anzahl von einen Boden des Kanals (4) mit der zweiten Seite (3) der Kanalplatte (1) durch einen Fließweg verbindenden ersten Durchflussöffnungen (5),
- eine Deckelplatte (15) mit einer ersten Seite (16) und einer zweiten Seite (17) und einer die zweite Seite (17) mit der ersten Seite (16) durch einen Fließweg verbindenden zweiten Durchflussöffnung (18), wobei die zweite Seite (17) der Deckelplatte (15) an der ersten Seite (2) der Kanalplatte (1) anliegt,
- eine Dichtungsplatte (11) mit einer ersten Seite (12) und einer zweiten Seite (13) und einer die erste Seite (12) mit der zweiten Seite (13) durch einen Fließweg verbindenden dritten Durchflussöffnung (14), wobei die erste Seite (12) der Dichtungsplatte (11) an der zweiten Seite (3) der Kanalplatte (1) anliegt,
wobei der Kanal (4) einen ersten Kanalabschnitt (9) zwischen einem Ende (6) des Kanals (4) und einer vorderen ersten Durchflussöffnung (7) durch die Kanalplatte (1) und eine Anzahl von sich in einer Fließrichtung oder entgegengesetzt einer Fließrichtung der Flüssigkeit hintereinander an den ersten Kanalabschnitt (9) anschließenden Verlängerungsabschnitten (10) zwischen der vorderen ersten Durchflussöffnung (7) und weiteren ersten Durchflussöffnungen (5) durch die Kanalplatte (1) aufweist.

2. Pumpe nach Anspruch 1,
wobei die Dichtungsplatte (11) bewegbar ist und mehrere Betriebsstellungen aufweist, wobei die dritte Durchflussöffnung (14) in jeder Betriebsstellung mit einer der ersten Durchflussöffnungen (5) in der Kanalplatte (1) fluchtet.

3. Pumpe nach Anspruch 1 oder 2,
wobei die dritte Durchflussöffnung (14) größer ist als die ersten Durchflussöffnungen (5).

4. Pumpe nach einem der Ansprüche 1 bis 3,
wobei die Kanalplatte (1) ein mikrostrukturierbares Glas aufweist.

5. Pumpe nach einem der Ansprüche 1 bis 4,
wobei die Kanalplatte (1) Silizium aufweist.

6. Pumpe nach einem der Ansprüche 1 bis 5,
wobei Oberflächen des Kanals (4), die mit der Flüssigkeit in Kontakt treten, ein gegenüber Lösungen mit einem pH-Wert von pH ≥ 10 resistentes Material aufweisen.

7. Pumpe nach einem der Ansprüche 1 bis 6,
wobei die Deckelplatte (15) aus Glas ausgebildet ist.

8. Pumpe nach einem der Ansprüche 1 bis 7,
wobei die Dichtungsplatte (11) aus Silikon ausgebildet ist.

9. Pumpe nach einem der Ansprüche 1 bis 8,
wobei die zweite Durchflussöffnung (18) in der Deckelplatte (15) in Fließrichtung der Flüssigkeit vor der dritten Durchflussöffnung (14) in der Dichtungsplatte (11) angeordnet ist, so dass durch einen Durchlass (22) in einem Verschluss (21) des Vorratsraums, durch die zweite Durchflussöffnung (18) in der Deckelplatte (15), durch den Kanal (4), durch eine erste Durchflussöffnung (5) in der Kanalplatte (1) und durch die dritte Durchflussöffnung (14) in der Dichtungsplatte (11) ein Fließweg für die Flüssigkeit gebildet ist.

10. Pumpe nach Anspruch 9,
wobei die erste Seite (16) der Deckelplatte (15) gegen den Verschluss (21) des Vorratsraums mittels eines O-Ringes (24) aus einem gummielastischen Material abgedichtet ist.

11. Pumpe nach einem der Ansprüche 1 bis 8,
wobei die zweite Durchflussöffnung (18) in der Deckelplatte (15) in Fließrichtung der Flüssigkeit nach der dritten Durchflussöffnung (14) in der Dichtungsplatte (11) angeordnet ist, so dass durch einen Einlass (22) in einem Verschluss (21) des Vorratsraums, durch die dritte Durchflussöffnung (14) in der Dichtungsplatte (11), durch eine erste Durchflussöffnung (5) in der Kanalplatte (1), durch den Kanal (4) und durch die zweite Durchflussöffnung (18) in der Deckelplatte (15) ein Fließweg für die Flüssigkeit gebildet ist.

12. Pumpe nach Anspruch 11,
wobei die erste Seite (16) der Deckelplatte (15) gegen das Gehäuse (19) mittels eines O-Ringes (24) aus einem gummielastischen Material abgedichtet ist.

13. Pumpe nach einem der Ansprüche 1 bis 12,
wobei die Infusionspumpe als in den Körper des Patienten implantierbare Infusionspumpe ausgebildet ist.

14. Verfahren zur Herstellung einer Kanalplatte (1) mit einer Drosselstrecke für eine Pumpe, wobei das Verfahren folgende Schritte aufweist:
- Bereitstellen einer Kanalplatte (1) mit einer ersten Seite (2) und einer zweiten Seite (3);
- Einbringen eines Kanals (4) in die erste Seite (2) der Kanalplatte (1);
- Einbringen von ersten Durchflussöffnungen (5) in die Kanalplatte (1), wobei die ersten Durchflussöffnungen (5) den Boden des Kanals (4) mit der zweiten Seite (3) der Kanalplatte (1) durch einen Fließweg verbinden;
- Aufbonden einer Deckelplatte (15) aus Glas mit ihrer zweiten Seite (17) auf die erste Seite (2) der Kanalplatte (1), wobei eine zweite Durchflussöffnung (18) in der Deckelplatte (15) mit einem Ende (6) des Kanals (4) fluchtet;
- Aufbringen einer Dichtungsplatte (11) mit ihrer ersten Seite (12) auf die zweite Seite (3) der Kanalplatte (1);
- Einsetzen der Kanalplatte (1) mit aufgebondeter Deckelplatte (15) und Dichtungsplatte (15) in ein Gehäuse (19).

15. Verfahren zur Inbetriebnahme einer Pumpe nach einem der Ansprüche 1 bis 13,
wobei eine Flussrate der Flüssigkeit durch den Kanal (4) dadurch eingestellt wird, dass eine der ersten Durchflussöffnungen (5) ausgewählt wird, für die der Fließweg der Flüssigkeit im Kanal (4) eine gewünschte Länge aufweist, und die dritte Durchflussöffnung (14) derart positioniert wird, dass sie mit der ausgewählten ersten Durchflussöffnung (8) fluchtet und die Dichtungsplatte (11) sämtliche anderen ersten Durchflussöffnungen (5) abdeckt.
